# EUROPEAN PATENT APPLICATION

(11) **EP 3 789 071 A1**
(43) Date of publication of application: **10.03.2021**
(21) Application number: 19195025.2
(22) Date of filing: 03.09.2019
(51) Int. Cl.: A61M 25/01

(54) **A MEDICAL DEVICE HANDLE FOR ARTICULATION OF A CATHETER**

(71) Applicant: Creganna Unlimited Company, Ballybrit, Galway H91 VN2T (IE)
(72) Inventor: MULDOON, Damian, Galway, H62 KPO3 (IE); MURPHY, Brian, Galway, H91V8XA (IE); WARD, Shane, Galway (IE); QUAID, Diarmuaid, Galway (IE)
(74) Representative: Grünecker Patent- und Rechtsanwälte PartG mbB

(57) **Abstract**

The present invention relates to a medical device handle (100) for articulation of a catheter (136) and a method of assembling same. The handle (100) comprises: a housing (107) having cylindrical shape, a control element (102) at least partly encompassed by the housing (107) and equipped with two threads (116) which are cross cut in opposing directions around a longitudinal axis of the handle (100), two pull wire mounting elements (112) each having a thread to engage with one of the cross cut threads of the control element (102), wherein the control element (102) is mechanically coupled to each of the pull wire mounting elements (112) via the threads without any additional gear in between, wherein a rotation of the control element (102) around the longitudinal axis causes the pull wire mounting elements (112) to move in opposite linear directions along the longitudinal axis.

## Description

The present invention relates to a medical device handle for articulation of a catheter. The present disclosure further relates to a method of assembling a medical device handle and to a method of actuating the handle.

A number of medical procedures require accessing and visualizing interior regions of a body lumen, for example of the heart. To that end, imaging systems based on a catheter can be used. Furthermore, it is known that intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional open surgery is a heart valve replacement or repair procedure in which an artificial valve or valve repair device is guided to the heart through the patient's vasculature.

The distal end of the catheter may be deflected by one or more deflecting mechanisms, which can be achieved by a tension cable, or other mechanisms positioned inside the catheter. Precise control of the distal end of the catheter allows for more reliable and faster positioning of a medical device and/or implant and other improvements in the procedures. Apart from structural heart applications, the catheters are also used for minimally invasive procedures such as neurovascular, coronary, structural heart, peripheral vascular or endoscopic type procedures for gastrointestinal applications or other.

In particular, steering systems are known that cause a distal end of a catheter to move in response to an actuation on the proximal end. For instance, it is known for articulation of catheter distal sections to use steering wires or cables with one end attached proximal pull wire mounts, the other end anchored at the catheter distal end. As used herein, the terms "proximal" and "distal" are to be taken as relative to a user using the disclosed delivery devices. "Proximal" is to be understood as relatively closer to the user and "distal" is to be understood as relatively farther away from the user.

In order to allow for an accurate steering of the catheter tip, there is still a need for an actuating mechanism that can be fabricated in a particularly simple manner and provides an accurate actuation via the medical device handle.

This object is solved by the subject matter of the independent claims. Advantageous embodiments of the present invention are the subject matter of the dependent claims.

The present invention provides a medical device handle for articulation of a catheter wherein rotating a control element causes two pull wire mounting elements to move in opposite linear directions and wherein the control element is directly coupled to said pull wire mounting elements. The direct coupling allows for a particularly precise manual operation of the pull wire mechanism.

A medical device handle according to the present invention comprises a housing having cylindrical shape and a control element. The housing can have an elongate catheter extending therefrom. Moreover, the control element is at least partly encompassed by the housing and it is equipped with two threads which are cross cut in opposing directions around a longitudinal axis of the handle.

The control element may comprise a rotating shaft having two external threads which are cross cut in opposing directions or a rotating component having two internal threads which are cross cut in opposing directions. It is rotatable about the longitudinal axis of the handle up to 720 degrees or more. The rotation of the control element can be limited by a stop element. Such a stop element enhances the safety of the handle mechanism.

Furthermore, the medical device handle comprises two pull wire mounting elements each having a thread to engage with one of the cross cut threads of the control element. The control element is mechanically coupled to each of the pull wire mounting elements via the threads without any additional gear in between. Moreover, a rotation of the control element around the longitudinal axis causes the pull wire mounting elements to move in opposite linear directions along the longitudinal axis. The pull wire mounting elements can be connected to pull wires which allow for steering the tip of the catheter. Moreover, a position of the pull wire mounting elements can be selectively locked by a locking mechanism movable between a locked and an unlocked position.

The present invention also relates to a corresponding method of assembling a medical device handle for articulation of a catheter. This method comprises providing a control element having two threads which are cross cut in opposing directions around a longitudinal axis of the control element; providing two pull wire mounting elements, each having a thread to engage with one of the cross cut threads of the control element; and providing a cylindrically shaped housing. The housing is mounted such that it at least partly encompasses said elements. Moreover, the method comprises mechanically coupling the control element to each of the pull wire mounting elements via the threads without any additional gear in between.

The method can also comprise crosscutting the threads into the external surface of the control element or into the internal surface of the control element. Moreover, the method can comprise connecting the pull wire mounting elements to pull wires.

The handle allows for releasing tension of the pull wires and for minimizing the number of components necessary, such that the handle can be fabricated in a particularly cost-efficient way and is less error-prone. The advantage of this solution can be seen in providing a particularly simple, but at the same time accurate steering system.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form-individually or in different combinations-solutions according to the present invention. The following described embodiments thus can be considered either alone or in an arbitrary combination thereof. Further features and advantages will become apparent from the following more particular description of the various embodiments of the invention, as illustrated in the accompanying drawings, in which like references refer to like elements, and wherein:
- **FIG. 1**: is a schematic exploded diagram view of a medical device handle according to a first example of the present disclosure.
- **FIG. 2**: is a schematic diagram illustrating that rotating the shaft anticlockwise moves the pull wire mounting elements in opposite directions;
- **FIG. 3**: is a schematic diagram illustrating that rotating the shaft clockwise moves the pull wire mounting elements in interchanged opposite directions;
- **FIG. 4**: is a further schematic exploded view illustrating the medical device handle;
- **FIG. 5**: is a perspective view of a handle according to another advantageous embodiment;
- **FIG. 6**: is a schematic exploded view of the handle of Figure 5;
- **FIG. 7**: is a schematic diagram illustrating the control element with two internal threads which are cross cut in opposing directions;
- **FIG. 8**: is a schematic diagram illustrating that rotating the control element anticlockwise moves the pull wire mounting elements in opposite directions;
- **FIG. 9**: is a schematic diagram illustrating that rotating the control element clockwise moves the pull wire mounting elements in interchanged opposite directions.

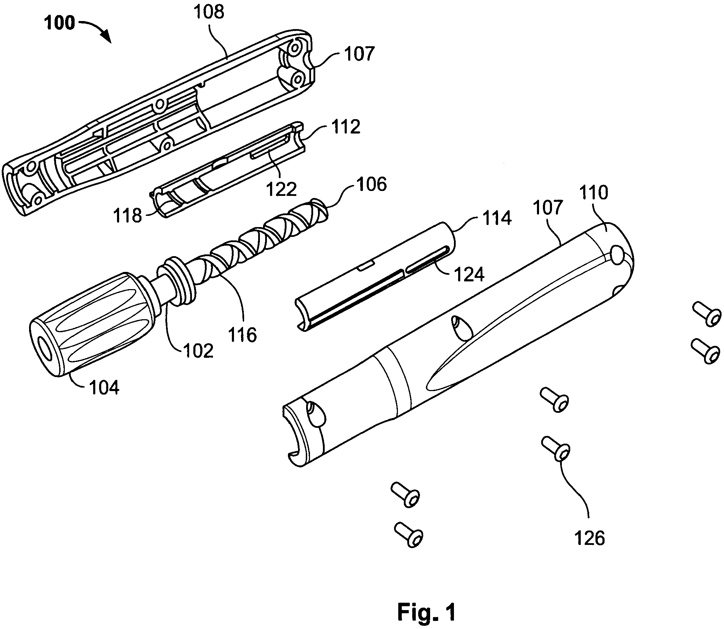

The present invention will now be explained in more detail with reference to the Figures and firstly referring to Figure 1, illustrating a rotating shaft having two external threads which are cross cut in opposing directions, whereas the pull wire mounting elements comprise internal threads.

Figure 1 shows an exploded view of a medical device handle 100 according to an exemplary embodiment of the present invention. The medical device handle 100 comprises a control element 102 with a rotatable adjustment knob 104 and a rotating shaft 106. The rotating shaft 106 has two external threads 116 which are cross cut in opposing directions. The medical device handle 100 can be attached to a proximal end of a catheter 136 for actuating pull wires which are attached to a distal end of the catheter 136. By rotating the adjustment knob 104 around a longitudinal axis of the handle 100, the pull wires can be actuated.

For attaching the pull wires, the medical device handle 100 further comprises a first and a second pull wire mounting element 112 and 114. The pull wire mounting elements 112 and 114 resemble the shape of a tube which is cut in two halves along its longitudinal axis. The pull wire mounting elements 112 and 114 surround the rotating shaft 106 of control element 102 from two sides. Each pull wire mounting element 112 and 114 has an internal thread 118 and 120 to suit one of the external crosscut threads 116 of the control element 102. The control element 102 is mechanically coupled to each of the pull wire mounting elements 112 and 114 via the threads without any additional gear in between. This reduces the number of components necessary such that the medical device handle 100 can be fabricated in a particular cost-efficient way and is less error-prone.

The medical device handle 100 further comprises a housing 107. The housing 107 at least partly encompasses the control element 102 and the pull wire mounting elements 112 and 114. The housing 107 comprises two halves 108 and 110 which surround the pull wire mounting elements 112 and 114. The two halves 108 and 110 are connected via screws 126. The housing 107 keeps the other elements of the medical device handle 100 together and serves as the actual handle to hold the medical device handle 100.

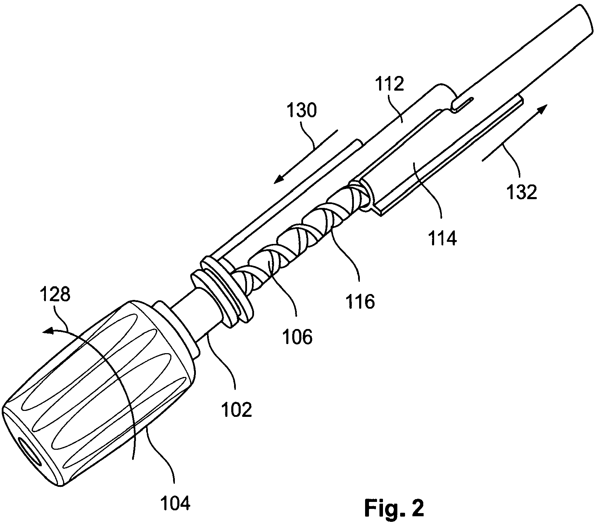

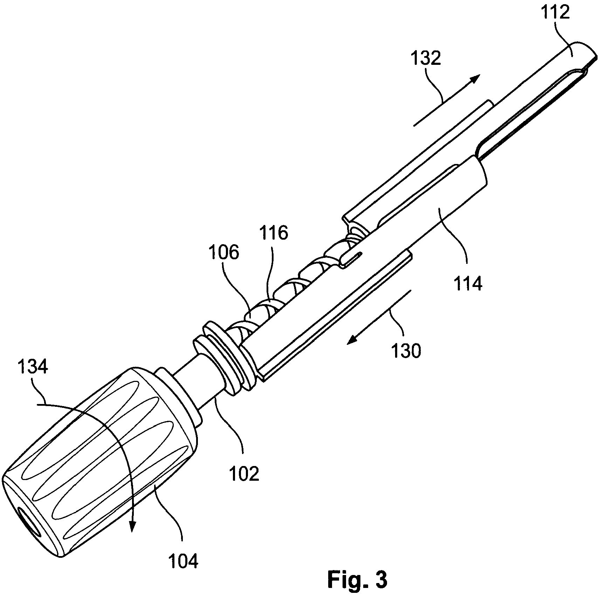

As shown in Figure 2, rotating the control element 102 in anticlockwise direction causes the pull wire mounting elements 112 and 114 to move in opposite linear directions 130 and 132 along the longitudinal axis of the medical device handle 100. As shown in Figure 3, rotating the control element 102 in clockwise direction causes the pull wire mounting elements 112 and 114 to move in interchanged opposite linear directions 132 and 130 along the longitudinal axis of the medical device handle 100.

It has to be noted that directions such as "left" and "right" and "clockwise" and "anticlockwise" refer to the standpoint of a person operating the device handle.

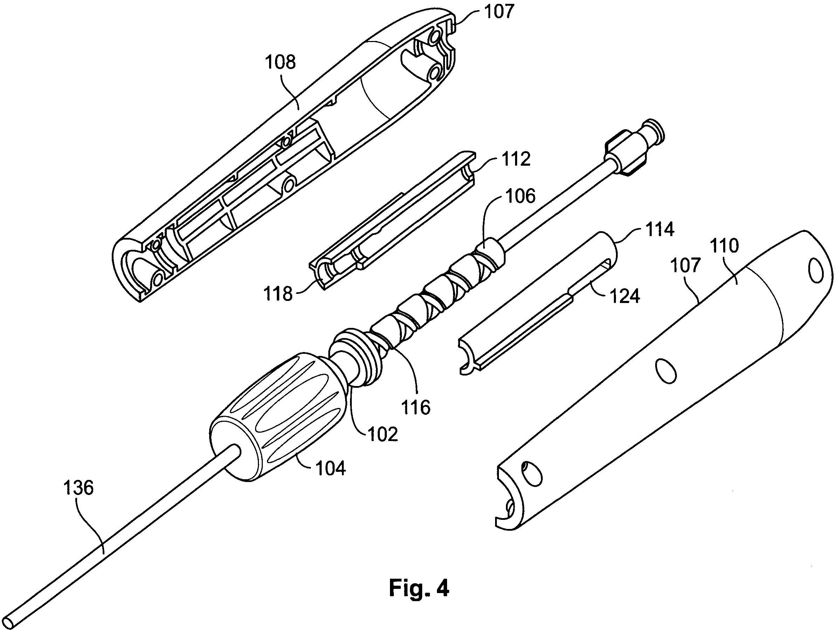

As shown in Figure 4, the medical device handle 100 can comprise an elongate catheter 136 which extends from the housing 107. The pull wire mounting elements 112 and 114 are connected to the catheter 136 via pull wires (not shown in the Figures). Accordingly, the position of the catheter 136 can be controlled by rotating the control element 102. Since the pull wire mounting elements 112 and 114 are moved in opposite linear directions, the tension of the longer side of the pull wires can be reduced.

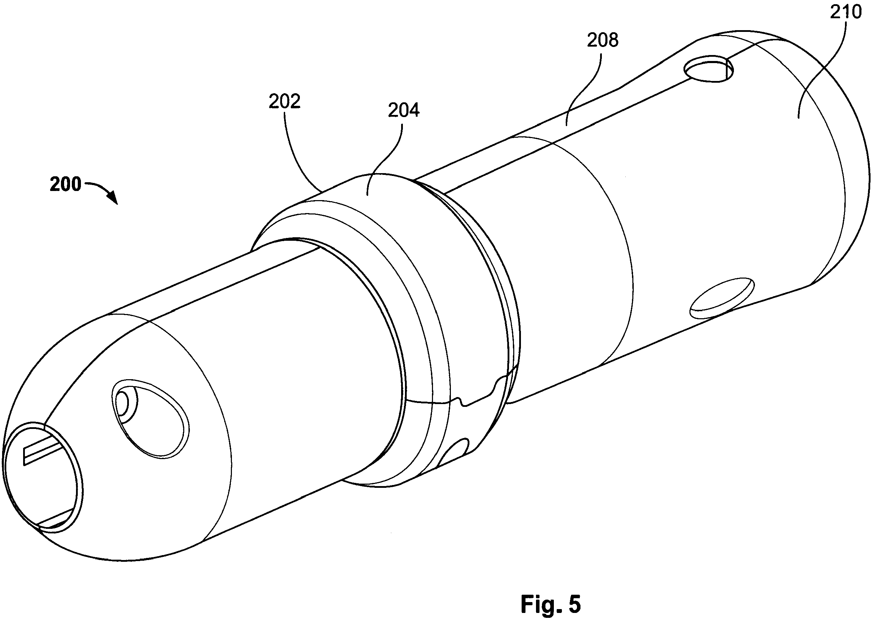

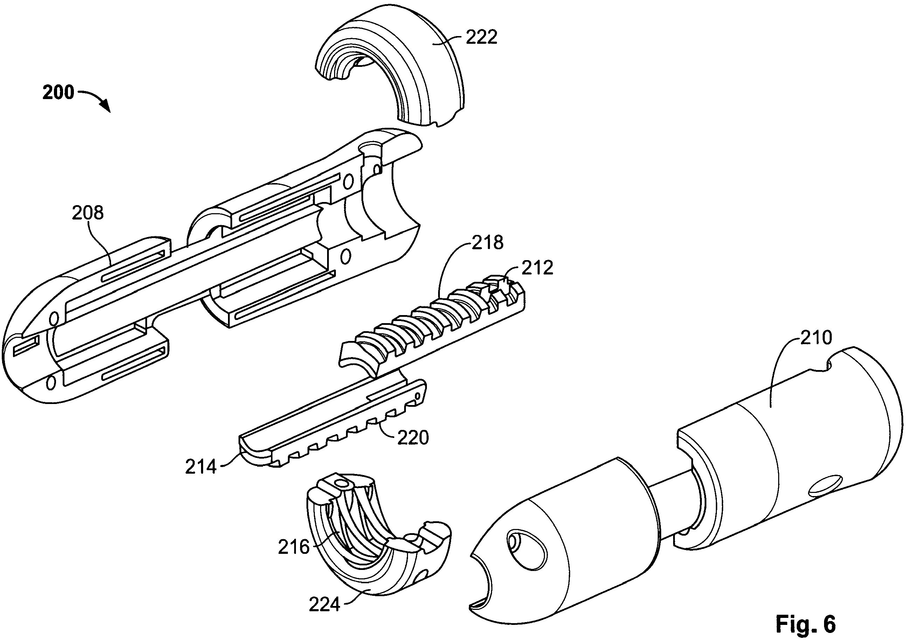

Another advantageous embodiment of the present invention is shown in Figures 5 and 6. This embodiment also relates to a medical device handle 200 comprising a control element having two threads which are cross cut in opposing directions, and pull wire mounting elements having a thread to suit one of the crosscut threads, and a housing.

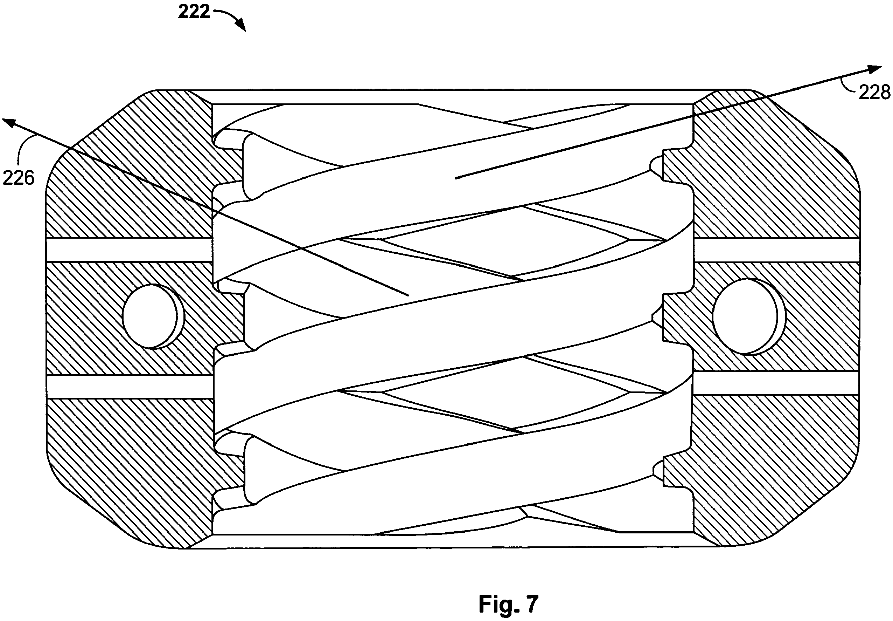

However, the control element 202 according to this embodiment has internal instead of external threads (c.f. Figures 6 and 7), whereas the pull wire mounting elements 212 and 214 have external instead of internal threads (c.f. Figure 6).

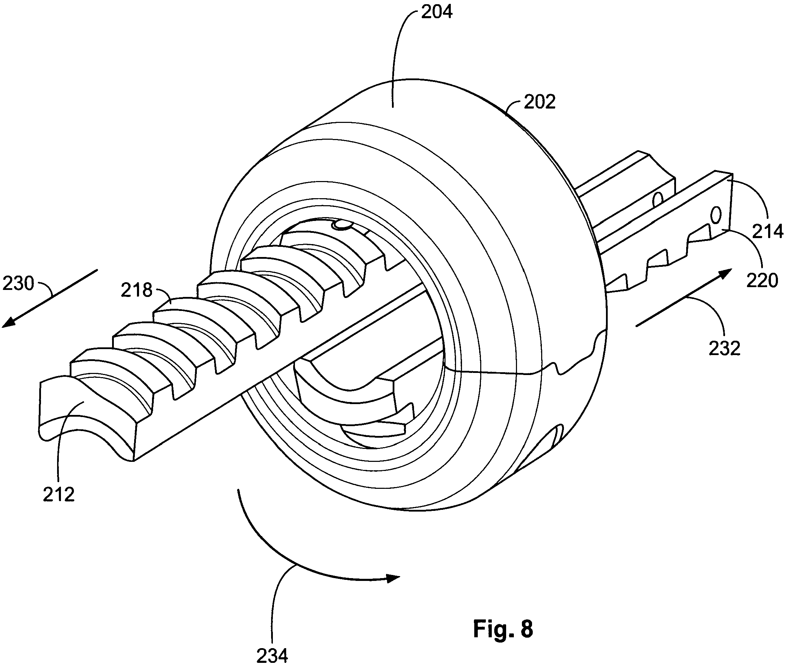

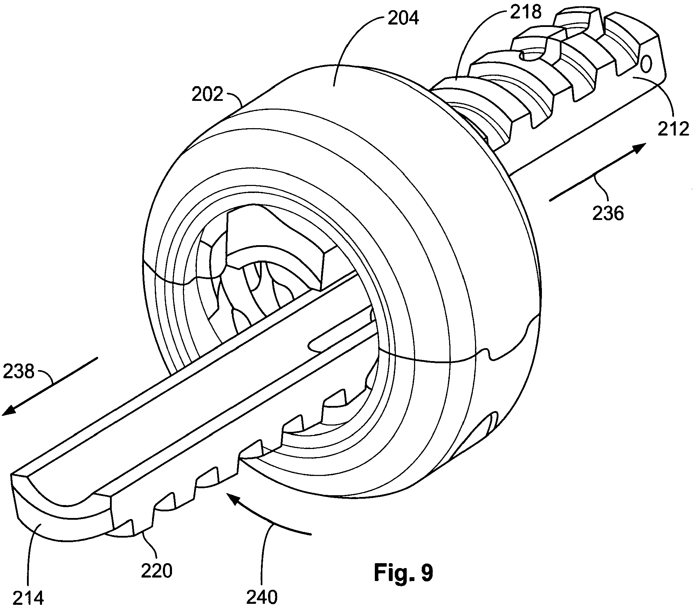

As shown in Figure 8, rotating the adjustment knob 204 anticlockwise causes the pull wire mounting elements 212 and 214 to move in opposite linear directions 230 and 232 along the longitudinal axis of the medical device handle 200. As shown in Figure 9, rotating the adjustment knob 204 clockwise causes the pull wire mounting elements 212 and 214 to move in interchanged opposite linear directions 232 and 230 along the longitudinal axis of the medical device handle 100. Therefore, the tension of the longer side of the pull wires can be reduced. Again, there is no additional gear between the control element 102 and the pull wire mounting elements 212 and 214, which reduces the number of components necessary such that the medical device handle 100 can be fabricated in a particular cost-efficient way and is less error-prone.

**REFERENCE NUMERALS**

| **Reference Numeral** | **Description** |
|---|---|
| 100, 200 | Medical device handle |
| 102, 202 | Control element |
| 104 | Adjustment knob of the control element |
| 106 | Rotatable shaft of the control element |
| 107 | Housing |
| 108 | First half shell of the housing |
| 110 | Second half shell of the housing |
| 112 | First pull wire mounting element with internal thread |
| 114 | Second pull wire mounting element with internal thread |
| 116 | External threads crosscut clockwise and anticlockwise |
| 118 | Internal thread of the first pull wire mounting element |
| 120 | Internal thread of the second pull wire mounting element |
| 122 | Connector for connecting a pull wire to the first pull wire mounting element |
| 124 | Connector for connecting a pull wire to the second pull wire mounting element |
| 126 | Screws |
| 128 | Rotation to the left |
| 130 | First linear direction |
| 132 | Second linear direction |
| 134 | Rotation to the right |
| 136 | Catheter |
| 204 | Adjustment knob with internal threads |
| 208 | First half shell of the housing in the second embodiment |
| 210 | Second half shell of the housing in the second embodiment |
| 212 | First pull wire mounting element with external thread |
| 214 | Second pull wire mounting element with external thread |
| 216 | Internal threads crosscut clockwise and anticlockwise |
| 218 | External thread of the first pull wire mounting element |
| 220 | External thread of the second pull wire mounting element |
| 222 | First half of the adjustment knob with internal threads |
| 224 | Second half of the adjustment knob with internal threads |
| 226 | Clockwise direction of the first internal thread |
| 228 | Anticlockwise direction of the second internal thread |
| 230 | Linear forward direction of the first pull wire mounting element |
| 232 | Linear backward direction of the second pull wire mounting element |
| 234 | Anticlockwise rotation of the control element |
| 236 | Linear backward direction of the first pull wire mounting element |
| 238 | Linear forward direction of the second pull wire mounting element |
| 240 | Clockwise rotation of the control element |

## Claims

1. A medical device handle for articulation of a catheter (136), the handle (100, 200) comprising:
a housing (107) having cylindrical shape,
a control element (102, 202) at least partly encompassed by the housing (107) and equipped with two threads (116, 216) which are cross cut in opposing directions around a longitudinal axis of the handle (100, 200),
two pull wire mounting elements (112, 212) each having a thread to engage with one of the cross cut threads (116, 216) of the control element (102, 202),
wherein the control element (102, 202) is mechanically coupled to each of the pull wire mounting elements (112, 212) via the threads without any additional gear in between,
wherein a rotation of the control element (102, 202) around the longitudinal axis causes the pull wire mounting elements (112, 212) to move in opposite linear directions along the longitudinal axis.

2. The medical device handle (100, 200) of claim 1, wherein the housing (107) has an elongate catheter (136) extending therefrom.

3. The medical device handle (100) according to claim 1 or 2, wherein the control element (102) comprises a rotating shaft having two external threads (116) which are cross cut in opposing directions.

4. The medical device handle (200) according to claim 1 or 2, wherein the control element (202) comprises a rotating component having two internal threads (216) which are cross cut in opposing directions.

5. The medical device handle (100, 200) according to any of the previous claims, wherein the pull wire mounting elements (112, 212) are connectable to pull wires which allow for steering the tip of the catheter (136).

6. The medical device handle (100, 200) according to any of the previous claims, wherein the control element (102, 202) is rotatable about the longitudinal axis of the handle.

7. The medical device handle (100, 200) according to any of the previous claims, further comprising a stop element which limits rotation of the control element.

8. The medical device handle (100, 200) according to any of the previous claims, wherein the control element (102, 202) is rotatable up to 720 degrees or more.

9. The medical device handle (100, 200) according to any of the previous claims, further comprising a locking mechanism movable between a locked and unlocked position which selectively locks a position of the pull wire mounting elements (112, 212).

10. Method of assembling a medical device handle (100, 200) for articulation of a catheter (136), the method comprising:
providing a control element (102, 202) having two threads (116, 216) which are cross cut in opposing directions around a longitudinal axis of the control element (102, 202),
providing two pull wire mounting elements (112, 212), each having a thread to engage with one of the cross cut threads of the control element (102, 202),
providing a cylindrically shaped housing (107),
mounting the housing (107) so that it at least partly encompasses said pull wire mounting elements and said control element, and
mechanically coupling the control element (102, 202) to each of the pull wire mounting elements (112) via the threads without any additional gear in between.

11. The method of claim 10, wherein the threads are cross cut into the external surface of the control element (102).

12. The method of claim 10, wherein the threads are cross cut into the internal surface of the control element (202).

13. The method according to any of the claims 10 to 12, comprising connecting pull wire mounting elements (112, 212) to pull wires.

14. Method of actuating the handle (100, 200) of any of the claims 1 to 9, wherein rotating the control element (102, 202) around its longitudinal axis causes the pull wire mounting elements (112, 212) to move in opposite linear directions.
